# EUROPÄISCHE PATENTSCHRIFT

(11) **EP 1 095 014 B1**
(45) Veröffentlichungstag und Bekanntmachung des Hinweises auf die Patenterteilung: **18.06.2003**
(21) Anmeldenummer: 99932735.6
(22) Anmeldetag: 26.06.1999
(51) Int. Cl.: C07C 251/60, A01N 37/50

(54) **METHOXYIMINO-PHENYLACETAMID-DERIVATE UND DEREN VERWENDUNG ALS SCHÄDLINGSBEKÄMPFUNGSMITTEL**
METHOXYMINO-PHENYLACETAMIDE DERIVATIVES AND THEIR UTILIZATION AS PESTICIDES
DERIVES DE METHOXY-IMINO-PHENYLACETAMIDE ET LEUR UTILISATION COMME PESTICIDES

(30) Priorität: 09.07.1998 DE 19830695
(43) Veröffentlichungstag der Anmeldung: 02.05.2001
(73) Patentinhaber: Bayer CropScience AG, 40789 Monheim (DE)
(72) Erfinder: HEINEMANN, Ulrich, D-42799 Leichlingen (DE); GAYER, Herbert, D-40789 Monheim (DE); GERDES, Peter, D-52080 Aachen (DE); VAUPEL, Martin, D-42799 Leichlingen (DE); MAULER-MACHNIK, Astrid, D-42799 Leichlingen (DE); HÄNSSLER, Gerd, D-51381 Leverkusen (DE); STENZEL, Klaus, D-40595 Düsseldorf (DE); KUGLER, Martin, D-42799 Leichlingen (DE); JAETSCH, Thomas, D-50668 Köln (DE); WACHTLER, Peter, D-47800 Krefeld (DE)
(86) Internationale Anmeldenummer: EP9904443
(87) Internationale Veröffentlichungsnummer: WO00002849

(56) Entgegenhaltungen:
- WO-A-97/00856

## Beschreibung

Die Erfindung betrifft neue Imide, mehrere Verfahren zu ihrer Herstellung und ihre Verwendung als Fungizide.

Es ist bekannt, daß bestimmte Imide, die den unten genannten ähnlich sind, fungizide Eigenschaften aufweisen (vgl. z. B. EP-A 398692, EP-A 528681 und WO 97-00856). Die Wirkung dieser Verbindungen ist jedoch insbesondere bei niedrigen Aufwandmengen nicht in allen Anwendungsgebieten völlig zufriedenstellend.

Es wurden nun die neuen Imide der allgemeinen Formel (I) gerunden, 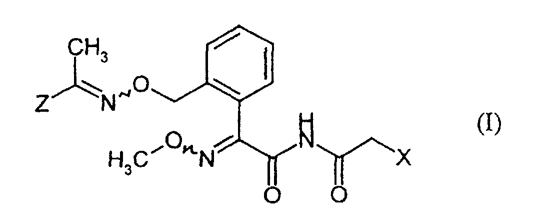 in welcher
- X: für Halogen oder Alkoxy steht und
- Z: für gegebenenfalls substituiertes Aryl oder für eine Gruppierung 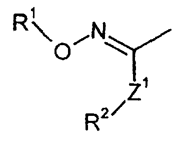 steht,
in welcher
R¹ für gegebenenfalls substituiertes Alkyl, Cycloalkyl oder Arylalkyl steht,
R² für gegebenenfalls substituiertes Alkyl, Aryl, Cycloalkyl, Arylalkyl, Heterocyclyl oder Heterocyclylalkyl steht,
Z¹ für eine Einfachbindung, Sauerstoff, Schwefel oder eine Gruppierung 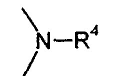 steht, wobei
R⁴ für Alkyl steht, oder gemeinsam mit R² und dem Stickstoffatom, an das sie gebunden sind, einen gegebenenfalls substituierten heterocyclischen Ring bildet.

Aryl steht für aromatische, mono- oder polycyclische Kohlenwasserstoffringe, wie beispielhaft und vorzugsweise Phenyl, Naphthyl, Anthranyl, Phenanthryl, vorzugsweise Phenyl oder Naphthyl, insbesondere Phenyl.

Weiterhin wurde gefunden, daß man die neuen substituierten Heterocycloalkene der allgemeinen Formel (I) erhält, wenn man
Carbonsäureamide der allgemeinen Formel (II) 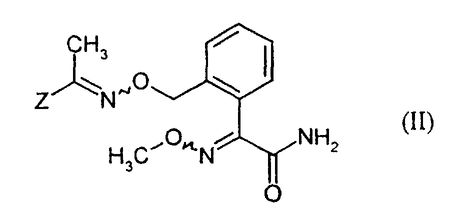 in welcher
- Z: die oben angegebene Bedeutung hat,
mit einem Carbonsäurehalogenid der Formel (III), 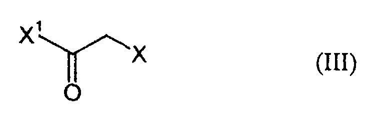 in welcher
- X: die oben angegebene Bedeutung hat, und
- X¹: für Halogen steht, oder
mit einem Carbonsäureanhydrid der Formel (IV), 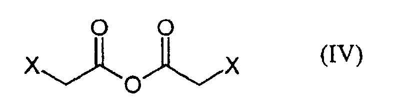 in welcher
- X: die oben angegebene Bedeutung hat,
jeweils gegebenenfalls in Gegenwart eines Verdünnungsmittels und jeweils gegebenenfalls in Gegenwart eines Säurebindemittels, umsetzt.

Schließlich wurde gefunden, daß die neuen Carbonsäureamidderivate der allgemeinen Formel (I) eine sehr starke füngizide Wirkung zeigen.

Die erfindungsgemäßen Verbindungen können gegebenenfalls als Mischungen verschiedener möglicher isomerer Formen, insbesondere von Stereoisomeren, wie z. B. E- und Z-Isomeren, gegebenenfalls aber auch von Tautomeren vorliegen. Es werden sowohl die E- als auch die Z-Isomeren, beliebige Mischungen dieser Isomeren, sowie die möglichen tautomeren Formen beansprucht.

Gegenstand der vorliegenden Anmeldung sind vorzugsweise Imide der Formel (I), in welcher
- X: für Chlor oder Alkoxy mit 1 bis 4 Kohlenstoffatomen steht und
- Z: für jeweils gegebenenfalls einfach oder mehrfach, gleich oder verschieden substituiertes Phenyl oder Naphthyl steht, wobei die möglichen Substituenten vorzugsweise aus der nachstehenden Aufzählung ausgewählt sind:
Halogen, Cyano, Nitro, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -
oder eine Gruppierung 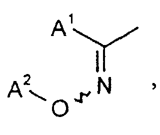 worin
- A¹: für Alkyl mit 1 bis 4 Kohlenstoffatomen oder Cycloalkyl mit 1 bis 6 Kohlenstoffatomen steht und
- A²: für gegebenenfalls durch Cyano, Alkoxy, Alkylthio, Alkylamino, Dialkylamino oder Phenyl substituiertes Alkyl mit 1 bis 4 Kohlentoffatomen, Alkenyl oder Alkinyl mit jeweils 2 bis 4 Kohlenstoffatomen steht,
- Z: ebenfalls für eine Gruppierung 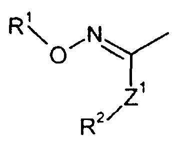 steht,
in welcher
R¹ für Alkyl mit 1 bis 12 Kohlenstoffatomen, gegebenenfalls durch Halogen oder Alkyl mit 1 bis 4 Kohlenstoffatomen substituiertes Cycloalkyl mit 3 bis 8 Kohlenstoffatomen oder gegebenenfalls im Arylteil substituiertes Arylalkyl mit 6 bis 10 Kohlenstoffatomen im Arylteil und 1 bis 4 Kohlenstoffatomen im Alkylteil steht, wobei die Substituenten vorzugsweise aus der nachfolgenden Aufzählung ausgewählt sind:
Halogen, Cyano, Nitro, jeweils geradkettiges oder verzweigtes Alkyl, Alkoxy, Alkoxyalkyl, Alkylthioalkyl, Alkylaminoalkyl, Dialkylaminoalkyl, Alkylthio, Alkylsulfinyl oder Alkylsulfonyl mit jeweils 1 bis 8 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;

R² fiir Alkyl mit 1 bis 12 Kohlenstoffatomen, gegebenenfalls durch Halogen oder Alkyl mit 1 bis 4 Kohlenstoffatomen substituiertes Cycloalkyl mit 3 bis 8 Kohlenstoffatomen,
für gegebenenfalls durch Halogen, Alkyl, Alkoxy, Alkylthio, Alkylsulfinyl oder Alkylsulfonyl mit 1 bis 4 Kohlenstoffatomen, Halogenalkoxy, Halogenalkylthio, Halogenalkylsulfinyl oder Halogenalkylsulfonyl mit 1 bis 4 Kohlenstoffatomen und 1 bis 9 Halogenatomen oder Phenyl, substituiertes Heterocyclyl, Benzoheterocyclyl, Dibenzoheterocyclyl oder Heterocyclylalkyl mit jeweils 3 bis 7 Ringgliedern im Heterocyclylteil und 1 bis 4 Kohlenstoffatomen im Alkylteil;
oder jeweils gegebenenfalls im Arylteil substituiertes Aryl oder Arylalkyl mit 6 bis 10 Kohlenstoffatomen im Arylteil und 1 bis 4 Kohlenstoffatomen im Alkylteil steht, wobei die Substituenten vorzugsweise aus der nachfolgenden Aufzählung ausgewählt sind:
Halogen, Cyano, Nitro, Amino, Carbamoyl, Thiocarbamoyl;
jeweils geradkettiges oder verzweigtes Alkyl, Alkoxy, Alkoxyalkyl, Alkylthioalkyl, Alkylaminoalkyl, Dialkylaminoalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkoxycarbonyl, Alkylaminocarbonyl, Dialkylaminocarbonyl oder Arylalkylaminocarbonyl mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
jeweils gegebenenfalls durch Halogen, Alkyl, Alkoxy, Alkylthio, Alkylsulfinyl oder Alkylsulfonyl mit 1 bis 4 Kohlenstoffatomen, Halogenalkoxy, Halogenalkylthio, Halogenalkylsulfinyl oder Halogenalkylsulfonyl mit 1 bis 4 Kohlenstoffatomen und 1 bis 9 Halogenatomen substituiertes Phenoxy oder Phenylalkoxy mit 1 bis 4 Kohlenstoffatomen im Alkylteil,
jeweils gegebenenfalls durch Halogen, Alkyl, Alkoxy, Alkylthio, Alkylsulfinyl oder Alkylsulfonyl mit 1 bis 4 Kohlenstoffatomen,
Halogenalkoxy, Halogenalkylthio, Halogenalkylsulfinyl oder Halogenalkylsulfonyl mit 1 bis 4 Kohlenstoffatomen und 1 bis 9 Halogenatomen oder Phenyl substituiertes Heterocyclyl, Heterocyclyloxy, Heterocyclylthio, Heterocyclylsulfinyl oder Heterocyclylsulfonyl mit 5 oder 6 Ringgliedern,
oder eine Gruppierung 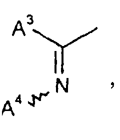 , worin
A³ für Wasserstoff oder Alkyl mit 1 bis 4 Kohlenstoffatomen oder Cycloalkyl mit 3 bis 6 Kohlenstoffatomen steht und
A⁴ für Hydroxy, Amino, Methylamino, Methyl, Phenyl, Benzyl, Alkoxy, Alkylamino, Dialkylamino mit 1 bis 4 Kohlenstoffatomen in den jeweiligen Alkylketten steht,
Z¹ für eine Einfachbindung, Sauerstoff, Schwefel oder eine Gruppierung 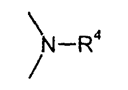 steht, wobei
R⁴ für Alkyl mit 1 bis 4 Kohlenstoffatomen steht, oder gemeinsam mit R² und dem Stickstoffatom, an das sie gebunden sind, einen gegebenenfalls substituierten, 3 bis 6 gliedrigen, heterocyclischen Ring bildet.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy oder Alkylthio, jeweils geradkettig oder verzweigt.

Halogenalkyl steht für teilweise oder vollständig halogeniertes Alkyl. Bei mehrfach halogeniertem Halogenalkyl können die Halogenatome gleich oder verschieden sein. Bevorzugte Halogenatome sind Fluor und Chlor und insbesondere Fluor. Trägt das Halogenalkyl noch weitere Substituenten, reduziert sich die maximal mögliche Zahl der Halogenatome auf die verbleibenden freien Valenzen.

Halogen steht im allgemeinen für Fluor, Chlor, Brom oder Iod, vorzugsweise für Fluor, Chlor oder Brom, insbesondere für Fluor oder Chlor.

Heterocyclyl steht für gesättigte oder ungesättigte, sowie aromatische, ringförmige Verbindungen, in denen mindestens ein Ringglied ein Heteroatom, d.h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Enthält der Ring mehrere Sauerstoffatome, stehen diese nicht benachbart. Gegebenenfalls bilden die ringförmigen Verbindungen mit weiteren carbocyclischen oder heterocyclischen, ankondensierten oder überbrückten Ringen gemeinsam ein polycyclisches Ringsystem. Ein polycyclisches Ringsystem kann über den heterocyclischen Ring oder einen ankondensierten carbocyciischen Ring verknüpft sein. Bevorzugt sind mono- oder bicyclische Ringsysteme, insbesondere mono- oder bicyclische, aromatische Ringsysteme.

Gegenstand der Erfindung sind insbesondere Imide der Formel (I), in welcher
- X: für Chlor, Methoxy, Ethoxy, n- oder i-Propyoxy, n-, i-, s- oder t-Butoxy steht und
- Z: für gegebenenfalls substituiertes insbesondere einfach oder zweifach, besonders bevorzugt in 3- und/oder 4-Stellung substituiertes Phenyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, 3-Fluorpropen-2-yloxy, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Propan-1,3-diyl, Butan-1,4-diyl, Methylendioxy oder Ethylendioxy,
oder eine Gruppierung 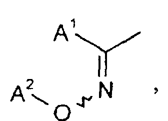 worin
A¹ insbesondere für Methyl, Ethyl, n- oder i-Propyl, n-, i-, s- oder t-Butyl, Cyclopropyl oder Cyclobutyl steht, und
A² insbesondere für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht,
- Z: ebenfalls für eine Gruppierung 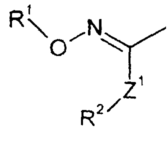 steht, in welcher
R¹ für jeweils gegebenenfalls einfach bis vierfach durch Fluor, Chlor, Methyl oder Ethyl substituiertes Cyclopentyl oder Cyclohexyl; oder für gegebenenfalls durch Methyl, Ethyl, Fluor, Chlor oder Brom substituiertes Cyclopropyl, Cyclobutyl, Cyclopentyl oder Cyclohexyl; oder für jeweils gegebenenfalls einfach bis vierfach, gleich oder verschieden substituiertes Benzyl, 1-Phenylethyl oder 2-Phenylethyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-. i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Methylaminomethyl, Dimethylaminomethyl,
Trifluormethyl, Trifluorethyl,
Difluormethoxy, Trifluormethoxy, Difluorchlormethoxy, Trifluorethoxy, Pentafluorethoxy, 2-Chlor-1,1,2-trifluorethoxy, Difluormethylthio, Trifluormethylthio, Difluorchlormethylthio, Trifluormethylsulfinyl oder Trifluormethylsulfonyl,

R² fiir Methyl, Ethyl, n- oder i-Propyl, n-, i-, s- oder t-Butyl, Pentyl, Hexyl, Heptyl, Octyl, Nonyl, Decyl, Undecyl, Dodecyl,
jeweils gegebenenfalls einfach bis vierfach durch Fluor, Chlor, Methyl oder Ethyl substituiertes Cyclopentyl oder Cyclohexyl; oder
für gegebenenfalls durch Methyl, Ethyl, Fluor, Chlor, Brom, Trifluormethyl, Phenyl substituiertes Thienyl, Pyridyl, Furyl, Piperazinyl, Thiazolyl, Dioxazinyl, Benzimidazolyl, Benzthiazolyl, Benzofuranyl, Benzopyrazolyl, Dibenzothiazinyl, Thienylmethyl, Pyridylmethyl oder Furylmethyl;
oder für jeweils gegebenenfalls im Phenylteil einfach bis vierfach, gleich oder verschieden substituiertes Phenyl, Benzyl, 1-Phenylethyl oder 2-Phenylethyl steht, wobei die möglichen Substituenten vorzugsweise aus der nachstehenden Aufzählung ausgewählt sind:
Fluor, Chlor, Brom, Cyano, Nitro, Amino, Carbamoyl, Thiocarbamoyl,
Methyl, Ethyl, n- oder i-Propyl, n-, i-, s- oder t-Butyl, Methoxymethyl,
Methoxy, Ethoxy, n- oder i-Propoxy,
Methylthio, Ethylthio, n- oder i-Propylthio, Methylsuifinyl, Ethylsulfinyl, Methylsulfonyl oder Ethylsulfonyl,
Methylaminomethyl, Dimethylaminomethyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Pentafluorethoxy, 2-Chlor-1,1,2-trifluorethoxy, Difluormethylthio, Trifluormethylthio, Difluorchlormethylthio, Trifluormethylsulfinyl oder Trifluormethylsulfonyl,
Methylamino, Ethylamino, n- oder i-Propylamino, Dimethylamino. Diethylamino,
Methoxycarbonyl, Ethoxycarbonyl, Methylaminocarbonyl, Ethylaminocarbonyl, Dimethylaminocarbonyl, Diethylaminocarbonyl, Cyclopentyl, Cyclohexyl, Cyclopentyloxy, Cyclohexyloxy,
jeweils gegebenenfalls einfach bis vierfach, gleich oder verschieden durch Fluor, Chlor, Methyl oder Trifluormethyl substituiertes, jeweils zweifach verknüpftes Propandiyl, Ethylenoxy, Methylendioxy, Ethylendioxy,
jeweils gegebenenfalls durch Fluor, Chlor, Methyl, Trifluormethyl oder Methoxy substituiertes Phenoxy oder Benzyl,
jeweils gegebenenfalls durch Halogen. Alkyl, Alkoxy, Alkylthio, Alkylsulfinyl oder Alkylsulfonyl mit 1 bis 4 Kohlenstoffatomen, Halogenalkoxy, Halogenalkylthio, Halogenalkylsulfinyl oder Halogenalkylsulfonyl mit 1 bis 4 Kohlenstoffatomen und 1 bis 9 Halogenatomen oder Phenyl substituiertes Heterocyclyl, Heterocyclyloxy, Heterocyclylthio, Heterocyclylsulfinyl oder Heterocyclylsulfonyl mit 5 oder 6 Ringgliedern,
für gegebenenfalls durch Methyl, Ethyl, Fluor, Chlor, Brom, Trifluormethyl, Phenyl substituiertes Thienyl, Imidazolyl, Thiadiazolyl, Pyridyl, Furyl, Piperazinyl, Thiazolyl, Dioxazinyl, Thiadiazolylsulfonyl;
   oder eine Gruppierung 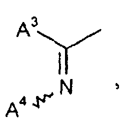 wobei
   A³ für Wasserstoff oder Methyl steht und
   A⁴ für Hydroxy, Methoxy, Ethoxy, Amino, Methylamino, Methyl, Phenyl oder Benzyl steht,

Z¹ für eine Einfachbindung, Sauerstoff, Schwefel oder eine Gruppierung 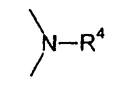 steht, wobei
R⁴ für Methyl, Ethyl, n- oder i-Propyl, n-, i-, s- oder t-Butyl steht, oder gemeinsam mit R² und dem Stickstoffatom, an das sie gebunden sind, einen gegebenenfalls substituierten, 5 bis 6 gliedrigen, heterocyclischen Ring bildet.
R¹ steht vorzugsweise für Ethyl und insbesondere für Methyl.
R² steht vorzugsweise für Methyl, Ethyl, n- oder i-Propyl, n-, i-, s- oder t-Butyl oder für einfach oder zweifach durch Fluor, Chlor, Methyl, Methoxy, Trifluormethyl und/oder Trifluormethoxy substituiertes Phenyl oder insbesondere für unsubstituiertes Phenyl.

Besonders bevorzugt sind Verbindungen, in denen X für Chlor, insbesondere für Methoxy steht.
- Z: steht bevorzugt für substituiertes oder unsubstituiertes Aryl, insbesondere Phenyl, wobei als Substituenten insbesondere Alkyl, Halogen, Halogenalkyl oder Halogenalkoxy bevorzugt sind.

Die Substituenten befinden sich in 2-, 3- und/oder insbesondere 4-Stellung.

Die oben aufgeführten allgemeinen oder in den Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Reste angegebenen Restedefinitionen werden unabhängig von der jeweilig angegebenen Kombination, beliebig auch durch Restedefinitionen anderer ersetzt. Außerdem können auch Restedefinitionen aus jedem Vorzugsbereich entfallen.

Die zur Durchführung des erfindungsgemäßen Verfahrens als Ausgangsstoffe benötigten Carbonsäureamide sind durch die Formel (II) allgemein definiert. In dieser Formel (II) haben X und Z vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für X und Z angegeben wurde.

Die Carbonsäureamide der Formel (II) sind bekannt und/oder können nach an sich bekannten Verfahren hergestellt werden (vgl. z. B. EP-A 398692 und EP-A 528681).
Die zur Durchführung des erfindungsgemäßen Verfahrens weiterhin als Ausgangsstoffe benötigten Carbonsäurehalogenide sind durch die Formel (III) allgemein definiert. In dieser Formel (III) hat X vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für X angegeben wurde. X¹ steht für Halogen, vorzugsweise für Chlor.

Die Carbonsäurehalogenide der Formel (III) sind bekannte Synthesechemikalien.

Die zur Durchführung des erfindungsgemäßen Verfahrens alternativ als Ausgangsstoffe benötigten Carbonsäureanhydride sind durch die Formel (IV) allgemein definiert. In dieser Formel (IV) hat X vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für X angegeben wurde.

Die Carbonsäureanhydride der Formel (IV) sind bekannte Synthesechemikalien.

Als Verdünnungsmittel zur Durchführung des erfindungsgemäßen Verfahrens kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methylt-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformarnid. N.N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid und Sulfone, wie Sulfolan.

Das erfindungsgemäße Verfahren wird gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen -20°C und 200°C, vorzugsweise bei Temperaturen zwischen 20°C und 180°C.

Zur Durchführung des erfindungsgemäßen Verfahrens zur Herstellung der Verbindungen der Formel (I) setzt man pro Mol des Carbonsäureamids der Formel (II) im allgemeinen 1 bis 15 Mol, vorzugsweise 1 bis 8 Mol an Carbonsäurehalogenid der Formel (III) oder Carbonsäureanhydrid der Formel (IV) ein.

Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach bekannten Verfahren (vgl. auch die Herstellungsbeispiele).

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorurn;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, Leptosphaeria-, Puccinia- oder Fusarium-Arten, von Krankheiten im Wein-, Obstund Gemüseanbau, wie beispielsweise gegen Venturia- und Plasmopara-Arten, oder von Reiskrankheiten, wie beispielsweise gegen Pyricularia-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Hoiz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Didobutrazol, Diclofluanid, Diclomezin, Dicloran. Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazole, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-ß-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid, 2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[{1-Methylethyl)-sulfonyl]-5-{trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis- 1-(4-Chlorphenyl)-2-( 1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide,
Chlorpyrifos, Chlozpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lamda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden.

Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

### Herstellungsbeispiele

### Beispiel (1)

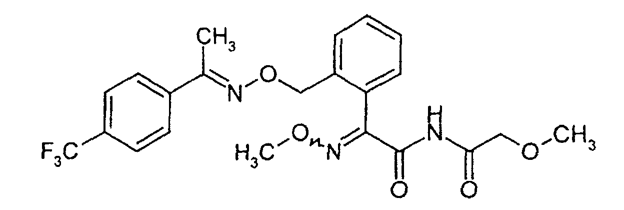

Eine Lösung aus 2,3 g (0,006 Mol) 2-Methoxyimino-2-{2-[l-(4-trifluormethylphenyl)-ethylidenaminooxymethyl]-phenyl}-acetamid und 0,6 g (0,0075 Mol) Pyridin in 20 ml Toluol wird auf ca. 90°C erhitzt. Hierzu tropft man bei dieser Temperatur eine Lösung von 6,5 g (0,06 Mol) Methoxyacetylchlorid in 20 ml Toluol und rührt 24 Stunden bei 90°C. Nach dem Abkühlen wird die Reaktionsmischung mit Wasser ausgeschüttelt. Die organische Phase wird abgetrennt, über Natriumsulfat getrocknet und im Vakuum eingeengt. Der Rückstand wird mit Cyclohexan/Essigester (9:1 bis 3:1) an Kieselgel chromatografiert. Man erhält 0.6 g (22% der Theorie) 2-Methoxy-N-(methoxyimino-{2-[1-(4-trifluormethylphenyl)-ethylidenaminooxymethyl]-phenyl}-acetyl)-acetamid vom Schmelzpunkt 134-138°C.

### Beispiel (2)

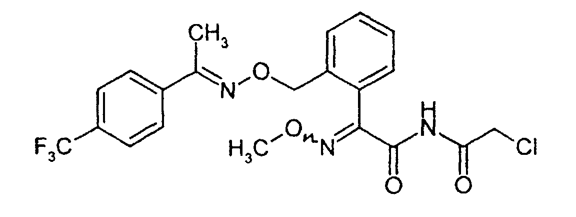

Eine Lösung aus 2,3 g (0,006 Mol) 2-Methoxyimino-2-{2-[1-(4-trifluormethylphenyl)-ethylidenaminooxymethyl]-phenyl}-acetamid und 0,6 g (0,0075 Mol) Pyridin in 20 ml Toluol wird auf ca. 90°C erhitzt. Hierzu tropft man bei dieser Temperatur eine Lösung von 6,8 g (0,06 Mol) Chloracetylchlorid in 20 ml Toluol und rührt 24 Stunden bei 90°C. Nach dem Abkühlen wird die Reaktionsmischung mit Wasser ausgeschüttelt. Die organische Phase wird abgetrennt, über Natriumsulfat getrocknet und im Vakuum eingeengt. Der Rückstand wird mit Cyclohexan/Essigester (19:1) an 3Kieselgel chromatografiert. Man erhält 0,3 g (11% der Theorie) 2-Chlor-N-(methoxyimino-{2-[1-(4-trifluormethyl-phenyl)-ethylidenaminooxymethyl]-phenyl}-acetyl)-acetamid vom Schmelzpunkt 92°C (Zers.).

Analog den Beispielen (1) und (2), sowie entsprechend der allgemeinen Beschreibung der erfindungsgemäßen Herstellungsverfahren, erhält man auch die in der nachstehenden Tabelle 1 aufgeführten crfindungsgemäßen Verbindungen der Formel (I):

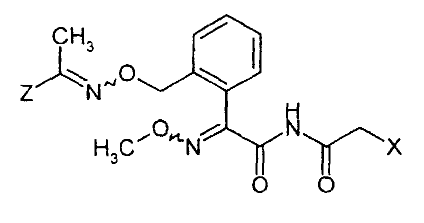

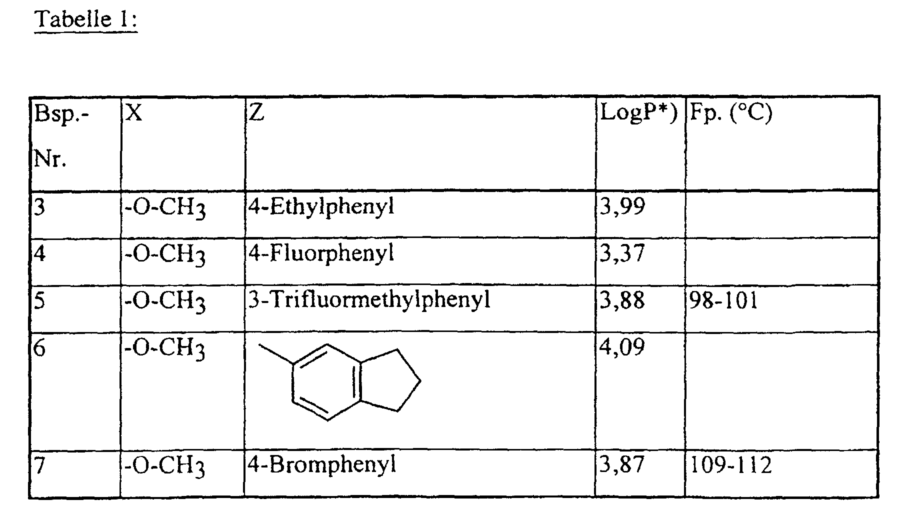

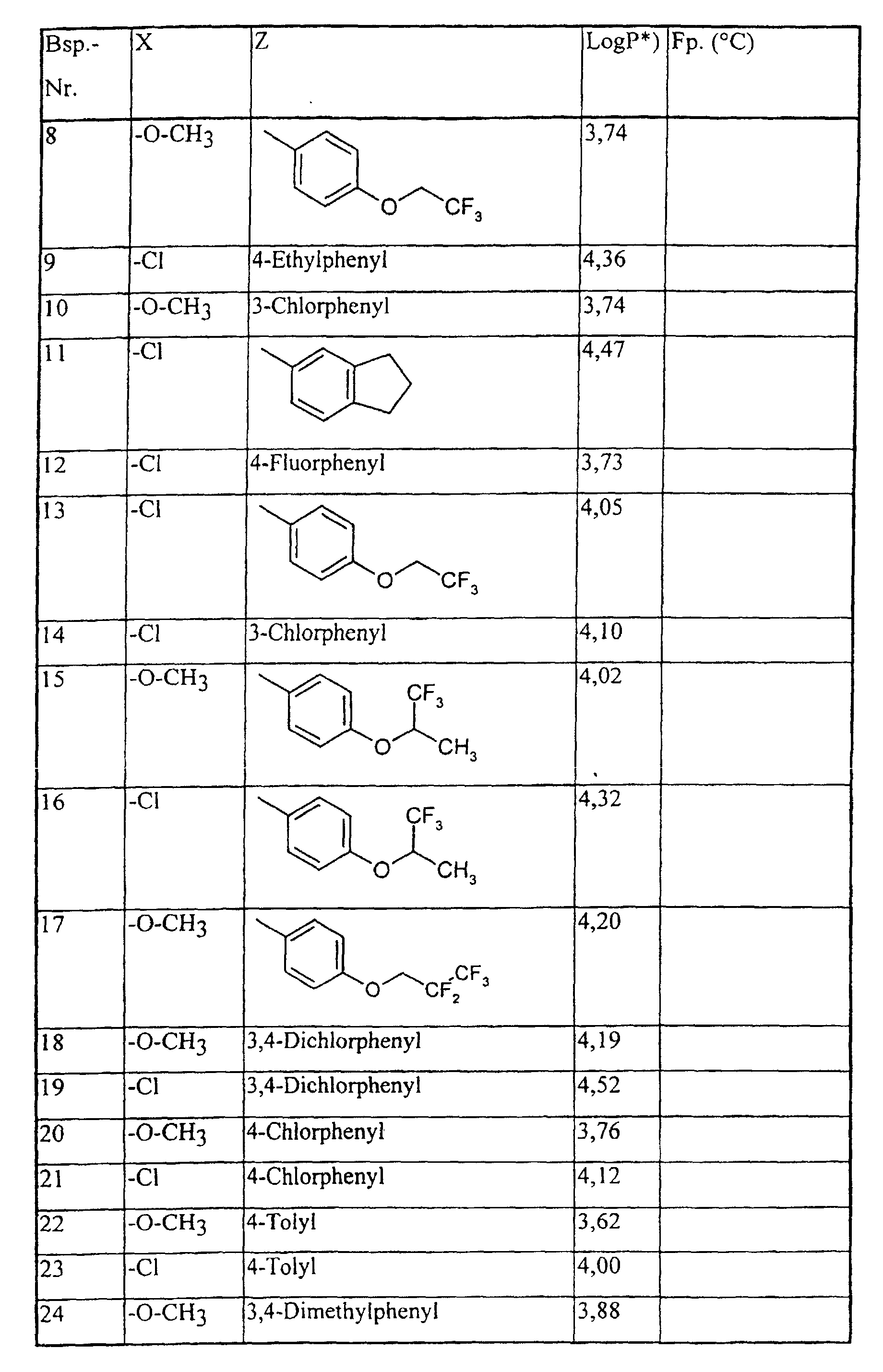

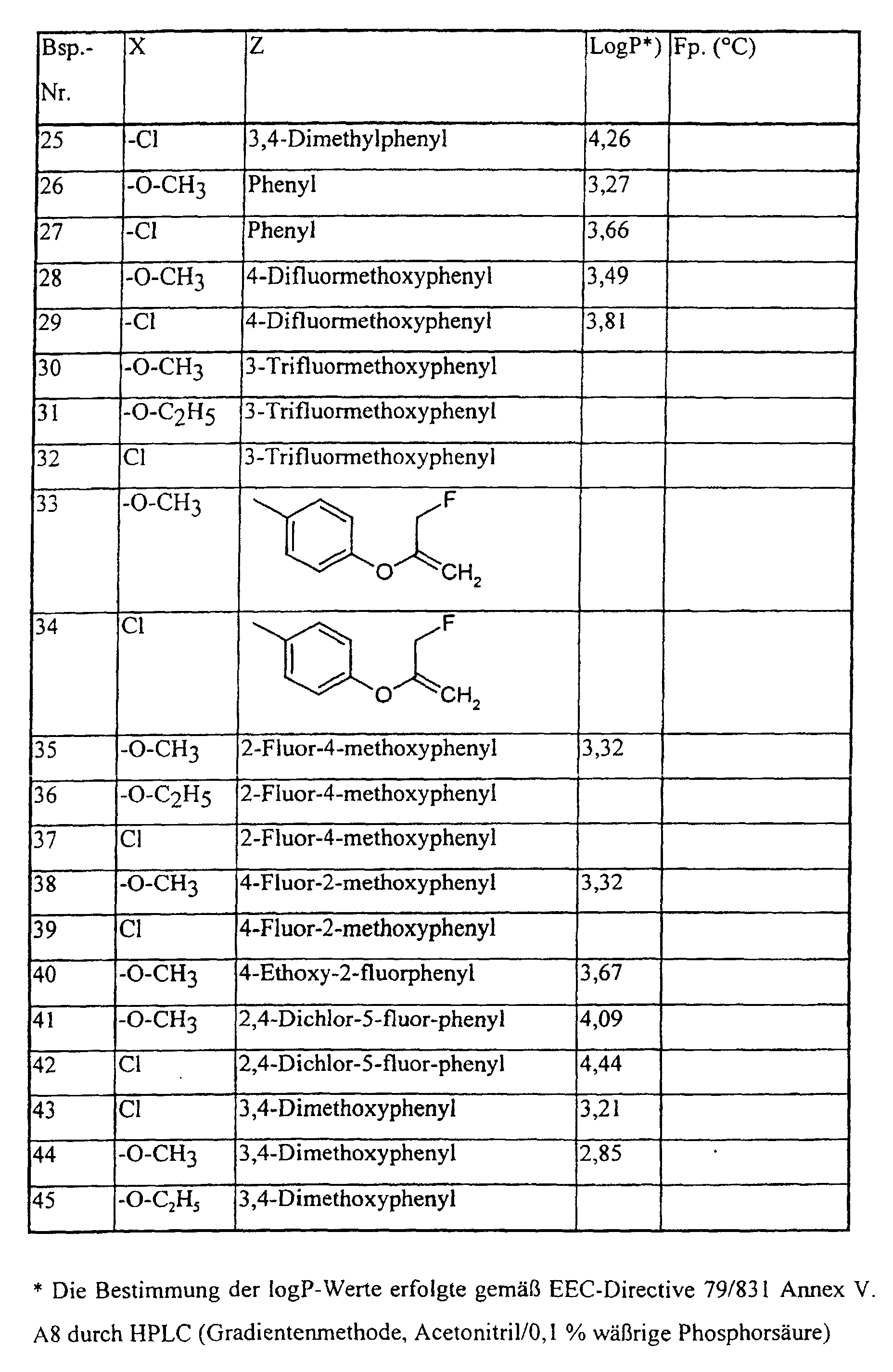

### Anwendungsbeispiele:

### Beispiel A

Fusarium nivale (var. nivale)-Test (Weizen) / protektiv

| | |
|---|---|
| Lösungsmittel | 25 Gewichtsteile N,N-Dimethylacetamid |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Fusarium nivale (var. nivale) besprüht.

Die Pflanzen werden in einem Gewächshaus unter lichtdurchlässigen Inkubationshauben bei einer Temperatur von ca. 15°C und einer relativen Luftfeuchtigkeit von ca. 100 % aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (10), (19) und (20) aufgeführten erfindungsgemäßen Stoffe bei einer Aufwandmenge von 250 g/ha einen Wirkungsgrad von 95 % oder mehr.

### Beispiel B

Erysiphe-Test (Gerste) / kurativ

| | |
|---|---|
| Lösungsmittel | 25 Gew.-Teile N,N-Dimethylactamid |
| Emulgator | 0,6 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigt der in dem Beispiel (14) aufgeführte erfindungsgemäß Stoff bei einer Aufwandmenge von 250 g/ha einen Wirkungsgrad von 95 % oder mehr.

### Beispiel C

Leptosphaeria nodorum-Test (Weizen) / protektiv

| | |
|---|---|
| Lösungsmittel | 25 Gewichtsteile N,N-Dimethylacetamid |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von 80 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (19), (20) und (21) aufgeführten erfindungsgemäßen Stoffe bei einer Aufwandmenge von 250 g/ha einen Wirkungsgrad von 95 % oder mehr.

### Beispiel D

Puccinia-Test (Weizen) / protektiv

| | |
|---|---|
| Lösungsmittel | 25 Gewichtsteile N,N-Dimethylacetamid |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (5), (19) und (22) aufgeführten erfindungsgemäßen Stoffe bei einer Aufwandmenge von 250 g/ha einen Wirkungsgrad von 95 % oder mehr.

### Beispiel E

Erysiphe-Test (Gerste) / protektiv

| | |
|---|---|
| Lösungsmittel | 25 Gew.-Teile N,N-Dimethylacetamid |
| Emulgator | 0,6 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbetages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (5), (13), (14), (15) und (18) aufgeführten erfindungsgemäßen Stoffe bei einer Aufwandmenge von 250 g/ha einen Wirkungsgrad von 100 % oder mehr.

### Beispiel F

Erysiphe-Test (Weizen) / protektiv

| | |
|---|---|
| Lösungsmittel | 25 Gew.-Teile N,N-Dimethylacetamid |
| Emulgator | 0,6 Gew.-Teile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigt der in dem Beispiel (5) aufgeführte erfindungsgemäße Stoff bei einer Aufwandmenge von 250 g/ha einen Wirkungsgrad von 95% oder mehr.

### Beispiel G

Fusarium graminearum-Test (Gerste) / protektiv

| | |
|---|---|
| Lösungsmittel | 25 Gewichtsteile N,N-Dimethylacetamid |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Fusarium graminearum besprüht.

Die Pflanzen werden in einem Gewächshaus unter lichtdurchlässigen Inkubationshauben bei einer Temperatur von ca. 15°C und einer relativen Luftfeuchtigkeit von 100 % aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigt der in dem Beispiel (8) aufgeführte erfindungsgemäße Stoff bei einer Aurwandmenge von 250g/ha einen Wirkungsgrad von 95 % oder mehr.

### Beispiel H

Plasmopara-Test (Rebe) / protektiv

| | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei ca. 20°C und 100 % relativer Luftfeuchtigkeit. Anschließend werden die Pflanzen 5 Tage im Gewächshaus bei ca. 21°C und ca. 90 % relativer Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (1), (3), (5), (9), (10), (11), (12), (13), (14), (15), (16), (18), (19), (20), (21), (25) und (27) aufgeführten erfindungsgemäßen Stoffe bei einer Aufwandmenge von 100 g/ha einen Wirkungsgrad von 90 % oder mehr.

### Beispiel I

Venturia-Test (Apfel) / protektiv

| | |
|---|---|
| Lösungsmittel | 47 Gewichtsteile Aceton |
| Emulgator | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers ***Venturia inaequalis*** inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (3), (6), (8), (9) und (11) aufgeführten erfindungsgemäßen Stoffe bei einer Aufwandmenge von 10 g/ha einen Wirkungsgrad von 95 % oder mehr.

### Beispiel K

Pyricularia-Test (Reis) / protektiv

| | |
|---|---|
| Lösungsmittel | 25 Gewichtsteile N,N-Dimethylacetamid |
| Emulgator | 0,06 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. Nach dem Antrocknen des Spritzbelages werden die Pflanzen mit einer wäßrigen Sporensuspension von Pyricularia oryzae inokuliert. Anschließend werden die Pflanzen in einem Gewächshaus bei 100 % relativer Luftfeuchtigkeit und 25°C aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die in den Beispielen (5), (7), (8), (9), (10), (11), (16), (18), (19), (20) und (22) aufgeführten erfindungsgemäßen Stoffe bei einer Aufwandmenge von 125 g/ha einen Wirkungsgrad von 70 % oder mehr.

## Patentansprüche

1. Verbindungen der Formel (I) 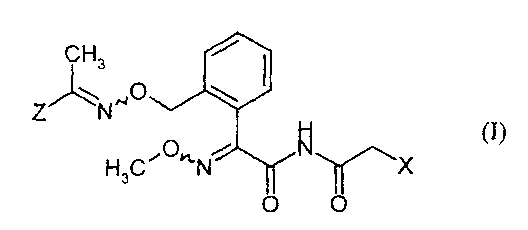 in welcher
X für Halogen oder Alkoxy steht und
Z für gegebenenfalls substituiertes Aryl oder für eine Gruppierung 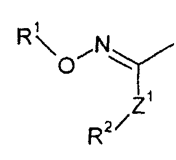 steht,
in welcher
R¹ für gegebenenfalls substituiertes Alkyl, Cycloalkyl oder Arylalkyl steht,
R² für gegebenenfalls substituiertes Alkyl, Aryl, Cycloalkyl, Arylalkyl, Heterocyclyl oder Heterocyclylalkyl steht,
Z¹ für eine Einfachbindung, Sauerstoff, Schwefel oder eine Gruppierung 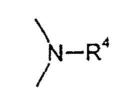 steht, wobei
R⁴ für Alkyl steht, oder gemeinsam mit R² und dem Stickstoffatom, an das sie gebunden sind, einen gegebenenfalls substituierten heterocyclischen Ring bildet.

2. Verbindungen der Formel (I) gemäß Anspruch 1, in welcher
X für Chlor oder Alkoxy mit 1 bis 4 Kohlenstoffatomen steht.

3. Verbindungen der Formel (I) gemäß Anspruch 1, in welcher
Z für jeweils gegebenenfalls einfach oder mehrfach. gleich oder verschieden substituiertes Phenyl oder Naphthyl steht, wobei die möglichen Substituenten vorzugsweise aus der nachstehenden Aufzählung ausgewählt sind:
Halogen, Cyano, Nitro, Hydroxy, Formyl, Carboxy, Carbamoyl. Thiocarbamoyl;
jeweils geradkettiges oder verzweigtes Alkyl, Alkoxy, Alkylthio, Alkysulfinyl oder Alkylsuifonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -
oder eine Gruppierung 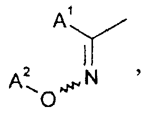 worin
A¹ für Alkyl mit 1 bis 4 Kohlenstoffatomen oder Cycloalkyl mit 1 bis 6 Kohlenstoffatomen steht und
A² für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht,
Z ebenfalls für eine Gruppierung 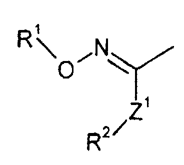 steht,
in welcher
R¹ für Alkyl mit 1 bis 12 Kohlenstoffatomen, gegebenenfalls durch Halogen oder Alkyl mit 1 bis 4 Kohlenstoffatomen substituiertes Cycloalkyl mit 3 bis 8 Kohlenstoffatomen oder gegebenenfalls im Arylteil substituiertes Arylalkyl mit 6 bis 10 Kohlenstoffatomen im Arylteil und 1 bis 4 Kohlenstoffatomen im Alkylteil steht, wobei die Substituenten aus der nachfolgenden Aufzählung ausgewählt sind:
Halogen, Cyano, Nitro, jeweils geradkettiges oder verzweigtes Alkyl, Alkoxy, Alkoxyalkyl, Alkylthioalkyl, Alkylaminoalkyl. Dialkylaminoalkyl, Alkylthio, Alkylsulfinyl oder Alkylsulfonyl mit jeweils 1 bis 8 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
R² für Alkyl mit 1 bis 12 Kohlenstoffatomen, gegebenenfalls durch Halogen oder Alkyl mit 1 bis 4 Kohlenstoffatomen substituiertes Cycloalkyl mit 3 bis 8 Kohlenstoffatomen,
für gegebenenfalls durch Halogen, Alkyl, Alkoxy, Alkylthio, Alkylsulfinyl oder Alkylsulfonyl mit 1 bis 4 Kohlenstoffatomen, Halogenalkoxy, Halogenalkylthio, Halogenalkylsulfinyl oder Halogenalkylsulfonyl mit 1 bis 4 Kohlenstoffatomen und 1 bis 9 Halogenatomen oder Phenyl, substituiertes Heterocyclyl, Benzoheterocyclyl, Dibenzoheterocyclyl oder Heterocyclylalkyl mit jeweils 3 bis 7 Ringgliedern im Heterocyclylteil und 1 bis 4 Kohlenstoffatomen im Alkylteil;
oder jeweils gegebenenfalls im Arylteil substituiertes Aryl oder Arylalkyl mit 6 bis 10 Kohlenstoffatomen im Arylteil und 1 bis 4 Kohlenstoffatomen im Alkylteil steht, wobei die Substituenten aus der nachfolgenden Aufzählung ausgewählt sind:
Halogen, Cyano, Nitro, Amino, Carbamoyl, Thiocarbamoyl;
jeweils geradkettiges oder verzweigtes Alkyl, Alkoxy, Alkoxyalkyl, Alkylthioalkyl, Alkylaminoalkyl, Dialkylaminoalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkoxycarbonyl, Alkylaminocarbonyl, Dialkylaminocarbonyl oder Arylalkylaminocarbonyl mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
jeweils gegebenenfalls durch Halogen, Alkyl, Alkoxy, Alkylthio, Alkylsulfinyl oder Alkylsulfonyl mit 1 bis 4 Kohlenstoffatomen, Halogenalkoxy, Halogenalkylthio, Halogenalkylsulfinyl oder Halogenalkylsulfonyl mit 1 bis 4 Kohlenstoffatomen und 1 bis 9 Halogenatomen substituiertes Phenoxy oder Phenylalkoxy mit 1 bis 4 Kohlenstoffatomen im Alkylteil, jeweils gegebenenfalls durch Halogen, Alkyl, Alkoxy, Alkylthio, Alkylsulfinyl oder Alkylsulfonyl mit 1 bis 4 Kohlenstoffatomen, Halogenalkoxy, Halogenalkylthio, Halogenalkylsulfinyl oder Halogenalkylsulfonyl mit 1 bis 4 Kohlenstoffatomen und 1 bis 9 Halogenatomen oder Phenyl substituiertes Heterocyclyl, Heterocyclyloxy, Heterocyclylthio, Heterocyclylsulfinyl oder Heterocyclylsulfonyl mit 5 oder 6 Ringgliedern,
oder eine Gruppierung 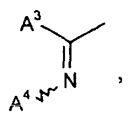 worin
A³ für Wasserstoff oder Alkyl mit 1 bis 4 Kohlenstoffatomen oder Cycloalkyl mit 3 bis 6 Kohlenstoffatomen steht und
A⁴ für Hydroxy, Amino, Methylamino, Methyl, Phenyl, Benzyl, Alkoxy, Alkylamino, Dialkylamino mit 1 bis 4 Kohlenstoffatomen in den jeweiligen Alkylketten steht,
Z¹ für eine Einfachbindung, Sauerstoff, Schwefel oder eine Gruppierung 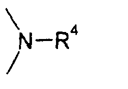 steht, wobei
R⁴ für Alkyl mit 1 bis 4 Kohlenstoffatomen steht, oder gemeinsam mit R² und dem Stickstoffatom, an das sie gebunden sind, einen gegebenenfalls substituierten, 3 bis 6 gliedrigen, heterocyclischen Ring bildet.

4. Verbindung der Formel (I), in welcher
X für Chlor, Methoxy, Ethoxy, n- oder i-Propoxy, n-, i-, s- oder t-Butoxy steht.

5. Verbindungen der Formel (I) gemäß Anspruch 1, in welcher
Z für gegebenenfalls substituiertes insbesondere einfach oder zweifach, besonders bevorzugt in 3- und/oder 4-Stellung substituiertes Phenyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, 3-Fluorpropen-2-yloxy, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Propan-1,3-diyl, Butan-1,4-diyl, Methylendioxy oder Ethylendioxy, oder eine Gruppierung 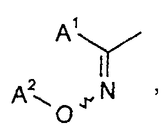 worin
A¹ insbesondere für Methyl, Ethyl, n- oder i-Propyl, n-, i-, s- oder t-Butyl, Cyclopropyl oder Cyclobutyl steht, und
A² insbesondere für Methyl, Ethyl, n- oder 1-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht,
Z ebenfalls fiir eine Gruppierung 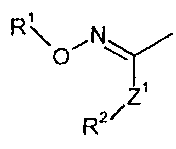 steht,
in welcher
R¹ für jeweils gegebenenfalls einfach bis vierfach durch Fluor, Chlor, Methyl oder Ethyl substituiertes Cyclopentyl oder Cyclohexyl; oder
für gegebenenfalls durch Methyl, Ethyl, Fluor, Chlor oder Brom substituiertes Cyclopropyl, Cyclobutyl, Cyclopentyl oder Cyclohexyl;
oder für jeweils gegebenenfalls einfach bis vierfach, gleich oder verschieden substituiertes Benzyl, 1-Phenylethyl oder 2-Phenylethyl steht, wobei die möglichen Substituenten aus der nachstehenden Aufzählung ausgewählt sind:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Methylaminomethyl, Dimethylaminomethyl,
Trifluormethyl, Trifluorethyl,
Difluormethoxy, Trifluormethoxy, Difluorchlormethoxy, Trifluorethoxy, Pentafluorethoxy, 2-Chlor-1,1,2-trifluorethoxy, Difluormethylthio, Trifluormethylthio, Difluorchlormethylthio, Trifluormethylsulfinyl oder Trifluormethylsulfonyl,
R² für Methyl, Ethyl, n- oder i-Propyl, n-, i-, s- oder t-Butyl, Pentyl, Hexyl, Heptyl, Octyl, Nonyl, Decyl, Undecyl, Dodecyl, jeweils gegebenenfalls einfach bis vierfach durch Fluor, Chlor, Methyl oder Ethyl substituiertes Cyclopentyl oder Cyclohexyl; oder
für jeweils gegebenenfalls durch Methyl, Ethyl, Fluor, Chlor, Brom, Trifluormethyl, Phenyl substituiertes Thienyl, Pyridyl, Furyl, Piperazinyl, Thiazolyl, Dioxazinyl, Benzimidazolyl, Benzthiazolyl, Benzofuranyl, Benzopyrazolyl, Dibenzothiazinyl, Thienylmethyl, Pyridylmethyl oder Furylmethyl;
oder für jeweils gegebenenfalls im Phenylteil einfach bis vierfach, gleich oder verschieden substituiertes Phenyl, Benzyl, 1-Phenylethyl oder 2-Phenylethyl steht, wobei die möglichen Substituenten aus der nachstehenden Aufzählung ausgewählt sind:
Fluor, Chlor, Brom, Cyano, Nitro, Amino, Carbamoyl, Thiocarbamoyl,
Methyl, Ethyl, n- oder i-Propyl, n-, i-, s- oder t-Butyl, Methoxymethyl,
Methoxy, Ethoxy, n- oder i-Propoxy,
Methylthio, Ethylthio, n- oder i-Propylthio, Methylsulfinyl, Ethylsulfinyl, Methylsulfonyl oder Ethylsulfonyl,
Methylaminomethyl, Dimethylaminomethyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Pentafluorethoxy, 2-Chlor-1,1,2-trifluorethoxy, Difluormethylthio, Trifluormethylthio, Difluorchlormethylthio, Trifluormethylsulfinyl oder Trifluormethylsulfonyl,
Methylamino, Ethylamino, n- oder i-Propylamino, Dimethylamino, Diethylamino,
Methoxycarbonyl, Ethoxycarbonyl, Methylaminocarbonyl, Ethylaminocarbonyl, Dimethylaminocarbonyl, Diethylaminocarbonyl,
Cyclopentyl, Cyclohexyl, Cyclopentyloxy, Cyclohexyloxy,
jeweils gegebenenfalls einfach bis vierfach, gleich oder verschieden durch Fluor, Chlor, Methyl oder Trifluormethyl substituiertes, jeweils zweifach verknüpftes Propandiyl, Ethylenoxy, Methylendioxy, Ethylendioxy
jeweils gegebenenfalls durch Fluor, Chlor, Methyl, Trifluormethyl oder Methoxy substituiertes Phenoxy oder Benzyl,
jeweils gegebenenfalls durch Halogen, Alkyl, Alkoxy, Alkylthio, Alkylsulfinyl oder Alkylsulfonyl mit 1 bis 4 Kohlenstoffatomen, Halogenalkoxy, Halogenalkylthio, Halogenalkylsulfinyl oder Halogenalkylsulfonyl mit 1 bis 4 Kohlenstoffatomen und 1 bis 9 Halogenatomen oder Phenyl substituiertes Heterocyclyl, Heterocyclyloxy, Heterocyclylthio, Heterocyclylsulfinyl oder Heterocyclylsulfonyl mit 5 oder 6 Ringgliedern,
für gegebenenfalls durch Methyl, Ethyl, Fluor, Chlor, Brom, Trifluormethyl, Phenyl substituiertes Thienyl, Imidazolyl, Thiadiazolyl, Pyridyl, Furyl, Piperazinyl, Thiazolyl, Dioxazinyl, Thiadiazolylsulfonyl; oder eine Gruppierung 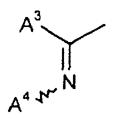 wobei
A³ für Wasserstoff oder Methyl steht und
A⁴ für Hydroxy, Methoxy, Ethoxy, Amino, Methylamino. Methyl, Phenyl oder Benzyl steht,
Z¹ für eine Einfachbindung, Sauerstoff, Schwefel oder eine Gruppierung 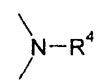 steht, wobei
R⁴ für Methyl, Ethyl, n- oder i-Propyl, n-, i-, s- oder t-Butyl steht. oder gemeinsam mit R² und dem Stickstoffatom, an das sie gebunden sind, einen gegebenenfalls substituierten, 5 bis 6 gliedrigen, heterocyclischen Ring bildet.

6. Mittel enthaltend Streckmittel und/oder Trägerstoffe sowie gegebenenfalls oberflächenaktive Stoffe, **gekennzeichnet durch** einen Gehalt an mindestens einer Verbindung der Formel (I) nach Anspruch 1.

7. Verfahren zur Bekämpfung von Schädlingen, **dadurch gekennzeichnet, daß** man Verbindungen der Formel (I) nach Anspruch 1 bzw. Mittel nach Anspruch 6 auf Schädlinge und/oder ihren Lebensraum einwirken läßt.

8. Verwendung von Verbindungen der Formel (I) nach den Ansprüchen 1 bis 5 bzw. Mittel nach Anspruch 6 zur Bekämpfung von Schädlingen.

9. Verfahren zur Herstellung von Mitteln, **dadurch gekennzeichnet, daß** man Verbindungen der Formel (I) nach den Ansprüchen 1 bis 5 mit Streckmitteln und/oder Trägerstoffen und/oder oberflächenaktiven Mitteln vermischt.

10. Verfahren zur Herstellung von Verbindungen der Formel (I) wie in Anspruch 1 definiert, **dadurch gekennzeichnet, daß** man Carbonsäureamide der allgemeinen Formel (II) 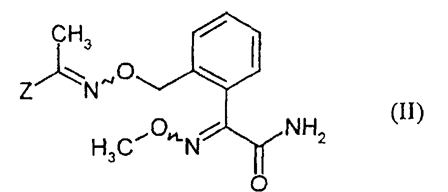 in welcher
Z die oben angegebene Bedeutung hat,
mit einem Carbonsäurehalogenid der Formel (III), 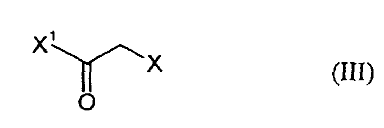 in welcher
X die oben angegebene Bedeutung hat, und
X¹ für Halogen steht, oder
mit einem Carbonsäureanhydrid der Formel (IV), 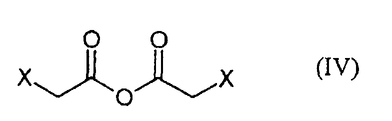 in welcher
X die oben angegebene Bedeutung hat,
jeweils gegebenenfalls in Gegenwart eines Verdünnungsmittels und jeweils gegebenenfalls in Gegenwart eines Säurebindemittels, umsetzt.

## Claims

1. Compounds of the formula (I) 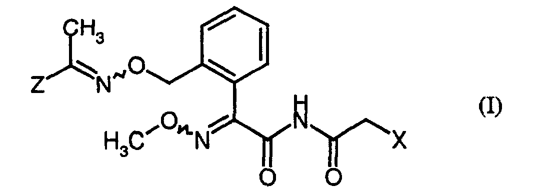 in which
X represents halogen or alkoxy and
Z represents optionally substituted aryl or a group 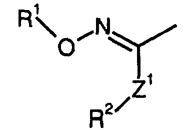 in which
R¹ represents optionally substituted alkyl, cycloalkyl or arylalkyl,
R² represents optionally substituted alkyl, aryl, cycloalkyl, arylalkyl, heterocyclyl or heterocyclylalkyl,
Z¹ represents a single bond, oxygen, sulphur or a group 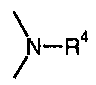 where
R⁴ represents alkyl, or together with R² and the nitrogen atom to which they are bonded forms an optionally substituted heterocyclic ring.

2. Compounds of the formula (I) according to Claim 1 in which
X represents chlorine or alkoxy having 1 to 4 carbon atoms.

3. Compounds of the formula (I) according to Claim 1 in which
Z represents phenyl or naphthyl, each of which is optionally monosubstituted or polysubstituted by identical or different substituents, the substituents which are possible preferably being selected from the list hereinbelow:
halogen, cyano, nitro, hydroxyl, formyl, carboxyl, carbamoyl, thiocarbamoyl;
in each case straight-chain or branched alkyl, alkoxy, alkylthio, alkylsulphinyl or alkylsulphonyl, each of which have 1 to 6 carbon atoms;
in each case straight-chain or branched alkenyl or alkenyloxy, each of which has 2 to 6 carbon atoms;
in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms;
in each case straight-chain or branched halogenoalkenyl or halogenoalkenyloxy each of which has 2 to 6 carbon atoms and 1 to 11 identical or different halogen atoms;
in each case straight-chain or branched alkylcarbonyl, alkylcarbonyloxy, alkoxycarbonyl or alkylsulphonyloxy, each of which has 1 to 6 carbon atoms in the individual alkyl moieties;
in each case divalent alkylene or dioxyalkylene, each of which has 1 to 6 carbon atoms and each of which is optionally monosubstituted or polysubstituted by identical or different substituents from the series consisting of halogen and/or straight-chain or branched alkyl having 1 to 4 carbon atoms and/or straight-chain or branched halogenoalkyl having 1 to 4 carbon atoms and 1 to 9 identical or different halogen atoms;
cycloalkyl having 3 to 6 carbon atoms;
heterocyclyl or heterocyclyl-methyl, each of which has 3 to 7 ring members of which in each case 1 to 3 are identical or different heteroatoms - in particular nitrogen, oxygen and/or sulphur -
or a group 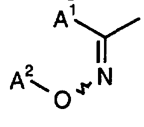 in which
A¹ represents alkyl with 1 to 4 carbon atoms or cycloalkyl having 1 to 6 carbon atoms and
A² represents alkyl having 1 to 4 carbon atoms, alkenyl or alkinyl, each of which has 2 to 4 carbon atoms, optionally substituted by cyano, alkoxy, alkylthio, alkylamino, dialkylamino or phenyl,
Z also represents a group 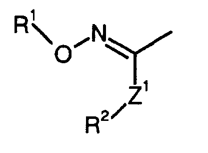 in which
R¹ represents alkyl having 1 to 12 carbon atoms, cycloalkyl having 3 to 8 carbon atoms which is optionally substituted by halogen or alkyl having 1 to 4 carbon atoms, or arylalkyl having 6 to 10 carbon atoms in the aryl moiety and 1 to 4 carbon atoms in the alkyl moiety, and which is optionally substituted in the aryl moiety, the substituents preferably being selected from the following list:
halogen, cyano, nitro, in each case straight-chain or branched alkyl, alkoxy, alkoxyalkyl, alkylthioalkyl, alkylaminoalkyl, dialkylaminoalkyl, alkylthio, alkylsulphinyl or alkylsulphonyl, each of which has 1 to 8 carbon atoms;
in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms;
R² represents alkyl having 1 to 12 carbon atoms, cycloalkyl having 3 to 8 carbon atoms which is optionally substituted by halogen or alkyl having 1 to 4 carbon atoms, or represents heterocyclyl, benzoheterocyclyl, dibenzoheterocyclyl or heterocyclylalkyl, each of which has 3 to 7 ring members in the heterocyclyl moiety and 1 to 4 carbon atoms in the alkyl moiety and each of which is optionally substituted by halogen, alkyl, alkoxy, alkylthio, alkylsulphinyl or alkylsulphonyl having 1 to 4 carbon atoms, halogenoalkoxy, halogenoalkylthio, halogenoalkylsulphinyl or halogenoalkylsulphonyl having 1 to 4 carbon atoms and 1 to 9 halogen atoms or phenyl;
or represents aryl or arylalkyl having 6 to 10 carbon atoms in the aryl moiety and 1 to 4 carbon atoms in the alkyl moiety, each of which is optionally substituted in the aryl moiety, the substituents preferably being selected from the following list:
halogen, cyano, nitro, amino, carbamoyl, thiocarbamoyl;
in each case straight-chain or branched alkyl, alkoxy, alkoxyalkyl, alkylthioalkyl, alkylaminoalkyl, dialkylaminoalkyl, alkylthio, alkylsulphinyl or alkylsulphonyl, each of which has 1 to 8 carbon atoms;
in each case straight-chain or branched alkenyl or alkenyloxy, each of which has 2 to 6 carbon atoms;
in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms;
in each case straight-chain or branched halogenoalkenyl or halogenoalkenyloxy each of which has 2 to 6 carbon atoms and 1 to 11 identical or different halogen atoms;
in each case straight-chain or branched alkylamino, dialkylamino, alkoxycarbonyl, alkylaminocarbonyl, dialkylaminocarbonyl or arylalkylaminocarbonyl having 1 to 6 carbon atoms in the respective hydrocarbon chains;
cycloalkyl or cycloalkyloxy, each of which has 3 to 6 carbon atoms;
in each case divalent alkylene having 3 or 4 carbon atoms, oxyalkylene having 2 or 3 carbon atoms or dioxyalkylene having 1 or 2 carbon atoms, in each case optionally monosubstituted to tetrasubstituted by identical or different substituents from the series consisting of fluorine, chlorine, methyl, trifluoromethyl or ethyl;
phenoxy or phenylalkoxy having 1 to 4 carbon atoms in the alkyl moiety and in each case optionally substituted by halogen, alkyl, alkoxy, alkylthio, alkylsulphinyl or alkylsulphonyl having 1 to 4 carbon atoms, halogenoalkoxy, halogenoalkylthio, halogenoalkylsulphinyl or halogenoalkylsulphonyl having 1 to 4 carbon atoms and 1 to 9 halogen atoms,
heterocyclyl, heterocyclyloxy, heterocyclylthio, heterocyclylsulphinyl or heterocyclylsulphonyl having 5 or 6 ring members, in each case optionally substituted by halogen, alkyl, alkoxy, alkylthio, alkylsulphinyl or alkylsulphonyl having 1 to 4 carbon atoms, halogenoalkoxy, halogenoalkylthio, halogenoalkylsulphinyl or halogenoalkylsulphonyl having 1 to 4 carbon atoms and 1 to 9 halogen atoms or phenyl,
or a group 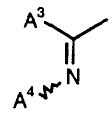 in which
A³ represents hydrogen or alkyl having 1 to 4 carbon atoms or cycloalkyl having 3 to 6 carbon atoms and
A⁴ represents hydroxyl, amino, methylamino, methyl, phenyl, benzyl, alkoxy, alkylamino, dialkylamino having 1 to 4 carbon atoms in the respective alkyl chains,
Z¹ represents a single bond, oxygen, sulphur or a group 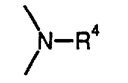 in which
R⁴ represents alkyl having 1 to 4 carbon atoms, or together with R² and the nitrogen atom to which they are bonded forms an optionally substituted, 3 to 6-membered, heterocyclic ring.

4. Compound of the formula (I) in which
X represents chlorine, methoxy, ethoxy, n- or i-propoxy, n-, i-, s- or t-butoxy.

5. Compounds of the formula (I) according to Claim 1 in which
Z represents phenyl which is optionally substituted, in particular monosubstituted or disubstituted, especially preferably substituted in the 3-and/or 4-position, the substituents which are possible preferably being selected from the following list:
fluorine, chlorine, bromine, cyano, nitro, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, 3-fluoropropen-2-yloxy, methoxycarbonyl, ethoxycarbonyl,
in each case divalent propane-1,3-diyl, butane-1,4-diyl, methylenedioxy or ethylenedioxy, each of which is optionally monosubstituted to tetrasubstituted by identical or different substituents from the series consisting of fluorine, chlorine, methyl, trifluoromethyl or ethyl, or a group 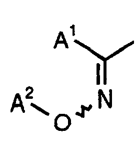 in which
A¹ represents, in particular, methyl, ethyl, n- or i-propyl, n-, i-, s- or t-butyl, cyclopropyl or cyclobutyl, and
A² represents, in particular, methyl, ethyl, n- or i-propyl, n-, i-, s- or t-butyl, allyl, propargyl, but-2-en-1-yl, 2-methyl-prop-1-en-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminomethyl, dimethylaminoethyl, methylaminomethyl, methylaminoethyl or benzyl,
Z likewise represents a group 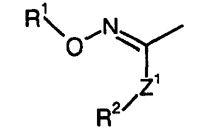 in which
R¹ represents cyclopentyl or cyclohexyl, each of which is optionally monosubstituted to tetrasubstituted by fluorine, chlorine, methyl or ethyl; or
represents cyclopropyl, cyclobutyl, cyclopentyl or cyclohexyl, optionally substituted by methyl, ethyl, fluorine, chlorine or bromine;
or represents benzyl, 1-phenylethyl or 2-phenylethyl, each of which is optionally monosubstituted to tetrasubstituted by identical or different substituents, the substituents which are possible preferably being selected from the following list:
fluorine, chlorine, bromine, cyano, nitro, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, methylaminomethyl, dimethylaminomethyl, trifluoromethyl, trifluoroethyl,
difluoromethoxy, trifluoromethoxy, difluorochloromethoxy, trifluoroethoxy, pentafluoroethoxy, 2-chloro-1,1,2-trifluoroethoxy, difluoromethylthio, trifluoromethylthio, difluorochloromethylthio, trifluoromethylsulphinyl or trifluoromethylsulphonyl,
R² represents methyl, ethyl, n- or i-propyl, n-, i-, s- or t-butyl, pentyl, hexyl, heptyl, octyl, nonyl, decyl, undecyl, dodecyl, cyclopentyl or cyclohexyl, each of which is optionally monosubstituted to tetrasubstituted by fluorine, chlorine, methyl or ethyl; or represents
thienyl, pyridyl, furyl, piperazinyl, thiazolyl, dioxazinyl, benzimidazolyl, benzothiazolyl, benzofuranyl, benzopyrazolyl, dibenzothiazinyl, thienylmethyl, pyridylmethyl or furylmethyl, each of which is optionally substituted by methyl, ethyl, fluorine, chlorine, bromine, trifluoromethyl, phenyl;
or represents phenyl, benzyl, 1-phenylethyl or 2-phenylethyl, each of which is optionally monosubstituted to tetrasubstituted in the phenyl moiety by identical or different substituents, the substituents which are possible preferably being selected from the following list:
fluorine, chlorine, bromine, cyano, nitro, amino, carbamoyl, thiocarbamoyl,
methyl, ethyl, n- or i-propyl, n-, i-, s- or t-butyl, methoxymethyl,
methoxy, ethoxy, n- or i-propoxy,
methylthio, ethylthio, n- or i-propylthio, methylsulphinyl, ethylsulphinyl, methylsulphonyl or ethylsulphonyl, methylaminomethyl, dimethylaminomethyl,
vinyl, allyl, 2-methylallyl, propen-1-yl, crotonyl, propargyl, vinyloxy, allyloxy, 2-methylallyloxy, propen-1-yloxy, crotonyloxy, propargyloxy; trifluoromethyl, trifluoroethyl,
difluoromethoxy, trifluoromethoxy, difluorochloromethoxy, trifluoroethoxy, pentafluoroethoxy, 2-chloro-1,1,2-trifluoroethoxy, difluoromethylthio, trifluoromethylthio, difluorochloromethylthio, trifluoromethylsulphinyl or trifluoromethylsulphonyl,
methylamino, ethylamino, n- or i-propylamino, dimethylamino, diethylamino,
methoxycarbonyl, ethoxycarbonyl, methylaminocarbonyl, ethylaminocarbonyl, dimethylaminocarbonyl, diethylaminocarbonyl,
cyclopentyl, cyclohexyl, cyclopentyloxy, cyclohexyloxy,
in each case divalent propanediyl, ethylenoxy, methylenedioxy, ethylenedioxy, each of which is optionally monosubstituted to tetrasubstituted by identical or different substituents from the series consisting of fluorine, chlorine, methyl or trifluoromethyl,
phenoxy or benzyl, each of which is optionally substituted by fluorine, chlorine, methyl, trifluoromethyl or methoxy,
heterocyclyl, heterocyclyloxy, heterocyclylthio, hetereocyclylsulphinyl or heterocyclylsulphonyl, each of which has 5 or 6 ring members and is optionally substituted by halogen, alkyl, alkoxy, alkylthio, alkylsulphinyl or alkylsulphonyl having 1 to 4 carbon atoms, halogenoalkoxy, halogenoalkylthio, halogenoalkylsulphinyl or halogenoalkylsulphonyl having 1 to 4 carbon atoms and 1 to 9 halogen atoms or phenyl,
or represents thienyl, imidazolyl, thiadiazolyl, pyridyl, furyl, piperazinyl, thiazolyl, dioxazinyl, thiadiazolylsulphonyl, optionally substituted by methyl, ethyl, fluorine, chlorine, bromine, trifluoromethyl, phenyl; or a group 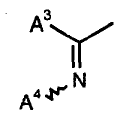 in which
A³ represents hydrogen or methyl and
A⁴ represents hydroxyl, methoxy, ethoxy, amino, methylamino, methyl, phenyl or benzyl,
Z¹ represents a single bond, oxygen, sulphur or a group 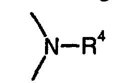 in which
R⁴ represents methyl, ethyl, n- or i-propyl, n-, i-, s- or t-butyl, or together with R² and the nitrogen atom to which they are bonded forms an optionally substituted 5 to 6-membered heterocyclic ring.

6. Compositions comprising extenders and/or carriers and, if appropriate, surfactants, **characterized by** a content of at least one compound of the formula (I) according to Claim 1.

7. Method of controlling pests, **characterized in that** compounds of the formula (I) according to Claim 1 or compositions according to Claim 6 are allowed to act on pests and/or their environment.

8. Use of compounds of the formula (I) according to Claims 1 to 5 or compositions according to Claim 6 for controlling pests.

9. Process for the preparation of compositions, **characterized in that** compounds of the formula (I) according to Claims 1 to 5 are mixed with extenders and/or carriers and/or surfactants.

10. Process for the preparation of compounds of the formula (I) as defined in Claim 1, **characterized in that** carboxamides of the formula (II) 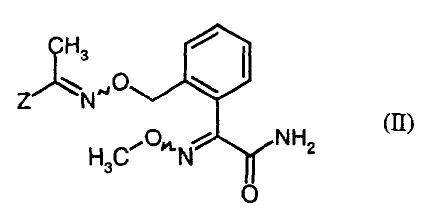 in which
Z is as defined above,
are reacted with an carbonyl halide of the formula (III), 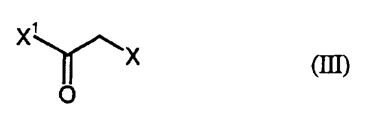 in which
X is as defined above, and
X¹ represents halogen, or
are reacted with a carboxylic anhydride of the formula (IV) 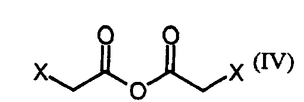 in which
X has the meaning given above,
in each case if appropriate in the presence of a diluent and in each case if appropriate in the presence of an acid binder.

## Revendications

1. Composés de formule (I) 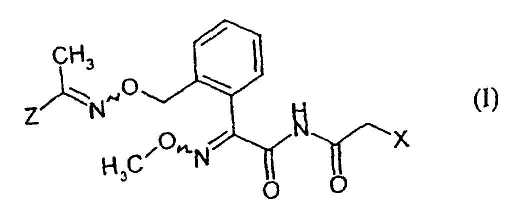 dans laquelle
X est un halogène ou un reste alkoxy et
Z représente un reste aryle éventuellement substitué ou un groupement de formule 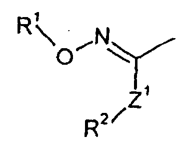 dans laquelle
R¹ est un reste alkyle éventuellement substitué, cycloalkyle ou arylalkyle,
R² est un reste alkyle éventuellement substitué, aryle, cycloalkyle, arylalkyle, hétérocyclyle ou hétérocyclylalkyle,
Z¹ représente une liaison simple, l'oxygène, le soufre ou un groupement 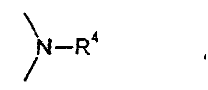 dans lequel
R⁴ représente un reste alkyle ou forme conjointement avec R² et l'atome d'azote auquel ils sont liés un noyau hétérocyclique éventuellement substitué.

2. Composés de formule (I) suivant la revendication 1, formule dans laquelle
X représente le chlore ou un reste alkoxy ayant 1 à 4 atomes de carbone.

3. Composés de formule (I) suivant la revendication 1, formule dans laquelle
Z représente un reste phényle ou naphtyle portant chacun, le cas échéant, un ou plusieurs substituants identiques ou différents, les substituants possibles étant choisis avantageusement dans l'énumération suivante :
halogène, cyano, nitro, hydroxy, formyle, carboxy, carbamoyle, thiocarbamoyle ;
alkyle, alkoxy, alkylthio, alkylsulfinyle ou alkylsulfonyle ayant chacun 1 à 6 atomes de carbone et chacun étant linéaire ou ramifié ;
un reste alcényle ou alcényloxy ayant chacun 2 à 6 atomes de carbone et chacun étant linéaire ou ramifié ;
un reste halogénalkyle, halogénlalkoxy, halogénalkylthio, halogénalkylsulfinyle ou halogénalkylsulfonyle ayant chacun 1 à 6 atomes de carbone et 1 à 13 atomes d'halogènes identiques ou différents et chacun étant linéaire ou ramifié ;
un reste halogénalcényle ou halogénalcényloxy ayant chacun 2 à 6 atomes de carbone et 1 à 11 atomes d'halogènes identiques ou différents et chacun étant linéaire ou ramifié ;
un reste alkylcarbonyle, alkylcarbonyloxy, alkoxycarbonyle ou alkylsulfonyloxy ayant chacun 1 à 6 atomes de carbone dans les parties alkyle individuelles et chacun étant linéaire ou ramifié ;
un reste alkylène ou dioxyalkylène ayant chacun 1 à 6 atomes de carbone, chacun deux liaison, et portant chacun, le cas échéant, un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
hétérocyclyle ou hétérocyclyl-méthyle chacun à noyau de 3 à 7 chaînons, dont 1 à 3 dans chaque cas sont des hétéroatomes identiques ou différents - en particulier azote, oxygène et/ou soufre -
ou un groupement 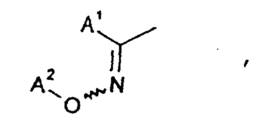 dans lequel
A¹ est un reste alkyle ayant 1 à 4 atomes de carbone ou cycloalkyle ayant 1 à 6 atomes de carbone et
A² est un reste alkyle ayant 1 à 4 atomes de carbone portant éventuellement un radical cyano, alkoxy, alkylthio, alkylamino, dialkylamino ou phényle, un reste alcényle ou un reste alcynyle ayant chacun 2 à 4 atomes de carbone,
Z représente également un groupement 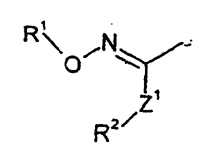 dans lequel
R¹ est un reste alkyle ayant 1 à 12 atomes de carbone, un reste cycloalkyle de 3 à 8 atomes de carbone portant éventuellement un substituant halogéno ou alkyle ayant 1 à 4 atomes de carbone,
ou un reste arylalkyle ayant 6 à 10 atomes de carbone dans la partie aryle et 1 à 4 atomes de carbone dans la partie alkyle et portant éventuellement un substituant dans la partie aryle, les substituants étant choisis parmi ceux qui sont énumérés ci-après :
halogène, cyano, nitro, alkyle, alkoxy, alkoxyalkyle, alkylthio-alkyle, alkylamino-alkyle, dialkylamino-alkyle, alkylthio, alkylsulfinyle ou alkylsulfonyle ayant chacun 1 à 8 atomes de carbone et chacun étant linéaire ou ramifié ; halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et chacun étant linéaire ou ramifié ;
R² est un reste alkyle ayant 1 à 12 atomes de carbone, un reste cycloalkyle de 3 à 8 atomes de carbone portant éventuellement un substituant halogéno ou alkyle ayant 1 à 4 atomes de carbone, un reste hétérocyclyle, benzohétérocyclyle, dibenzohétérocyclyle ou hétérocyclylalkyle ayant chacun un noyau de 3 à 7 chaînons dans la partie hétérocyclyle et 1 à 4 atomes de carbone dans la partie alkyle et portant éventuellement un substituant halogéno, alkyle, alkoxy, alkylthio, alkylsulfinyle ou alkylsulfonyle ayant 1 à 4 atomes de carbone, halogénalkoxy, halogénalkylthio, halogénalkylsulfinyle ou halogénalkylsulfonyle ayant 1 à 4 atomes de carbone et 1 à 9 atomes d'halogènes ou phényle ;
ou bien un reste aryle ou arylalkyle ayant 6 à 10 atomes de carbone dans la partie aryle et 1 à 4 atomes de carbone dans la partie alkyle et chacun étant éventuellement substitué dans la partie aryle, les substituants étant choisis parmi ceux qui sont énumérés ci-après :
halogène, cyano, nitro, amino, carbamoyle, thiocarbamoyie ;
alkyle, alkoxy, alkoxyalkyle, alkylthio-alkyle, alkylamino-alkyle, dialkylamino-alkyle, alkylthio, alkylsulfinyle ou alkylsulfonyle ayant chacun 1 à 8 atomes de carbone et chacun étant linéaire ou ramifié ;
alcényle ou alcényloxy ayant chacun 2 à 6 atomes de carbone et chacun étant linéaire ou ramifié ; halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et chacun étant linéaire ou ramifié ;
halogénalcényle ou halogénalcényloxy ayant chacun 2 à 6 atomes de carbone et 1 à 11 atomes d'halogènes identiques ou différents et chacun étant linéaire ou ramifié ;
alkylamino, dialkylamino, aikoxycarbonyle, alkylaminocarbonyle, dialkylaminocarbonyle ou arylalkylaminocarbonyle ayant 1 à 6 atomes de carbone dans les chaînes hydrocarbonées correspondantes et chacun étant linéaire ou ramifié ;
cycloalkyle ou cycloalkyloxy ayant chacun 3 à 6 atomes de carbone ;
alkylène ayant 3 ou 4 atomes de carbone, oxyalkylène ayant 2 à 3 atomes de carbone ou dioxyalkylène ayant 1 ou 2 atomes de carbone ayant chacun deux liaisons et chacun portant éventuellement un à quatre substituants, identiques ou différents, fluoro, chloro, méthyle, trifluorométhyle ou éthyle ;
phénoxy ou phénylalkoxy ayant 1 à 4 atomes de carbone dans la partie alkyle, portant chacun, le cas échéant, un substituant halogéno, alkyle, alkoxy, alkylthio, alkylsulfinyle ou alkylsulfonyle ayant 1 à 4 atomes de carbone, halogénalkoxy, halogénalkylthio, halogénalkylsulfinyle ou halogénalkylsulfonyle ayant 1 à 4 atomes de carbone et 1 à 9 atomes d'halogènes, hétérocyclyle, hétérocyclyloxy, hétérocyclylthio, hétérocycylylsulfinyle ou hétérocyclylsulfonyle pentagonal ou hexagonal portant chacun, le cas échéant, un substituant halogéno, alkyle, alkoxy, alkylthio, alkylsulfinyle ou alkylsulfonyle ayant 1 à 4 atomes de cabone, halogénalkoxy, halogénalkylthio, halogénalkylsulfinyle ou halogénalkylsulfonyle ayant 1 à 4 atomes de carbone et 1 à 9 atomes d'halogènes ou phényle,
ou bien un groupement F page 48 dans lequel
A₃ représente l'hydrogène ou un reste alkyle ayant 1 à 4 atomes de carbone ou cycloalkyle ayant 3 à 6 atomes de carbone et
A⁴ est un reste hydroxy, amino, méthylamino, méthyle, phényle, benzyle, alkoxy, alkylamino, dialkylamino ayant 1 à 4 atomes de carbone dans chacune des chaînes alkyle,
Z¹ représente une liaison simple, l'oxygène, le soufre ou un groupement 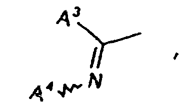 dans lequel
R⁴ est un reste alkyle ayant 1 à 4 atomes de carbone ou forme conjointement avec R² et l'atome de carbone auquel ils sont liés un noyau hétérocyclique de 3 à 6 chaînons, éventuellement substitué.

4. Composé de formule (I), dans laquelle
X représente le chlore, un reste méthoxy, éthoxy, n-propoxy, isopropoxy, n-butoxy, isobutoxy, sec.-butoxy ou tertio-butoxy.

5. Composés de formule (I) suivant la revendication 1, formule dans laquelle
Z est un reste phényle éventuellement substitué, portant en particulier un ou deux substituants, notamment en position 3 et/ou en position 4, les substituants possibles étant avantageusement choisis parmi ceux qui sont énumérés ci-après :
fluor, chlore, brome, cyano, nitro, méthyle, éthyle, n-propyle, isopropyle, n-butyle, iso-butyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, trifluorométhylthio, difluorochlorométhylthio, trifluorométhylsulfinyle, trifluorométhylsulfonyle, 3-fluoropropène-2-yloxy, méthoxycarbonyle, éthoxycarbonyle,
un reste propane-1,3-diyle, butane-1,4-diyle, méthylènedioxy ou éthylènedioxy ayant chacun deux liaisons et portant chacun, le cas échéant, un à quatre substituants, identiques ou différents, fluoro, chloro, méthyle, trifluorométhyle ou éthyle,
ou un groupement de formule 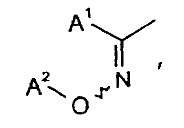 dans laquelle
A¹ représente en particulier un reste méthyle, éthyle, n-propyle, isopropyle, n-butyle, iso-butyle, sec.-butyle, tertio-butyle, cyclopropyle ou cyclobutyle, et
A² est en particulier un reste méthyle, éthyle, n-propyle, isopropyle, n-butyle, isobutyle, sec.-butyle, tertio-butyle, allyle, propargyle, but-2-ène-1-yle, 2-méthylprop-1-ène-3-yle, cyanométhyle, méthoxyméthyle, éthoxyméthyle, méthoxyéthyle, éthoxyéthyle, méthylthiométhyle, éthylthiométhyle, méthylthio-éthyle, éthylthioéthyle, diméthylaminométhyle, diméthylaminoéthyle, méthylaminométhyle, méthylamino-éthyle ou benzyle,
Z est également un groupement 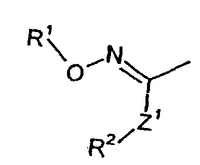 dans lequel
R¹ est un reste cyclopentyle ou cyclohexyle portant chacun, le cas échéant, un à quatre substituants fluoro, chloro, méthyle ou éthyle ;
ou bien
un reste cyclopropyle, cyclobutyle, cyclopentyle ou cyclohexyle portant éventuellement un substituant méthyle, éthyle, fluoro, chloro ou bromo ;
ou un reste benzyle, 1-phényléthyle ou 2-phényléthyle portant chacun, le cas échéant, un à quatre substituants identiques ou différents, les substituants possibles étant choisis parmi ceux qui sont énumérés ci-après :
fluor, chlore, bromoe, cyano, nitro, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, métylsulfonyle, éthylsulfonyle, méthylaminométhyle, diméthylaminométhyle, trifluorométhyle, trifluoréthyle, difluorométhoxy, trifluorométhoxy, difluorochlorométhoxy, trifluoréthoxy, pentafluoréthoxy, 2-chloro-1,1,2-trifluoréthoxy, difluorométhylthio, trifluorométhylthio, difluorochlorométhylthio, trifluorométhylsulfinyle ou trifluorométhylsulfonyle,
R² est un reste méthyle, éthyle, n-propyle, isopropyle, n-butyle, isobutyle, sec.-butyle, tertio-butyle, pentyle, hexyle, heptyle, octyle, nonyle, décyle, undécyle, dodécyle, un reste cyclopentyle ou cyclohexyle portant chacun, le cas échéant, un à quatre substituants fluoro, chloro, méthyle ou éthyle ;
ou bien
un reste thiényle, pyridyle, furyle, pipérazinyle, thiazolyle, dioxazinyle, benzimidazolyle, benzothiazolyle, benzofurannyle, benzopyrazolyle, dibenzothiazinyle, thiénylméthyle, pyridylméthyle ou furylméthyle portant chacun, le cas échéant, un substituant méthyle, éthyle, fluoro, chloro, bromo, trifluorométhyle, phényle ;
ou un reste phényle, benzyle, 1-phényléthyle ou 2-phényléthyle portant chacun, le cas échéant, dans la partie phényle un à quatre substituants identiques ou différents, les substituants possibles étant choisis parmi ceux qui sont énumérés ci-après : fluor, chlore, brome, cyano, nitro, amino, carbamoyle, thiocarbamoyle,
méthyle, éthyle, n-propyle, isopropyle, n-butyle, isobutyle, sec.-butyle, tertiobutyle,
méthoxyméthyle, méthoxy, éthoxy, n-propoxy ou isopropoxy, méthylthio, éthylthio, n-propylthio, isopropylthio, méthylsulfinyle, éthylsulfinyle, méthylsulfonyle ou éthylsulfonyle, méthylaminométhyle, diméthylaminométhyle, vinyle, allyle, 2-méthylallyle, propène-1-yle, crotonyle, propargyle, vinyloxy, allyloxy, 2-méthylallyloxy, propène-1-yloxy, crotonyloxy, propargyloxy ;
trifluorométhyle, trifluoréthyle, difluorométhoxy, trifluorométhoxy, difluorochlorométhoxy, trifluoréthoxy, pentafluoréthoxy, 2-chloro-1,1,2-trifluoréthoxy, difluorométhylthio, trifluorométhylthio, difluorochlorométhylthio, trifluorométhylsulfinyle ou trifluorométhylsulfonyle, méthylamino, éthylamino, n-propylamino, isopropylamino, diméthylamino, diéthylamino, méthoxycarbonyle, éthoxycarbonyle, méthylaminocarbonyle, éthylaminocarbonyle, diméthylaminocarbonyle, diéthylaminocarbonyle, cyclopentyle, cyclohexyle, cyclopentyloxy, cyclohexyloxy,
propanediyle, éthylèneoxy, méthylènedioxy, éthylènedioxy ayant chacun deux liaisons et portant chacun, le cas échéant, un à quatre substituants, identiques ou différents, fluoro, chloro, méthyle ou trifluorométhyle, phénoxy ou benzyle portant chacun, le cas échéant, un substituant fluoro, chloro, méthyle, tirfluorométhyle ou méthoxy,
hétérocyclyle, hétérocyclyloxy, hétérocyclylthio, hétérocyclylsulfinyle ou hétérocyclylsulfonyle à noyau pentagonal ou hexagonal, portant chacun, le cas échéant, un substituant halogéno, alkyle, alkoxy, alkylthio, alkylsulfinyle ou alkylsulfonyle ayant 1 à 4 atomes de carbone, halogénaikoxy, halogénalkylthio, halogénalkylsulfinyle ou halogénalkylsulfonyle ayant 1 à 4 atomes de carbone et 1 à 9 atomes d'halogènes identiques ou différents, ou phényle,
thiényle, imidazolyle, thiadiazolyle, pyridyle, furyle, pipérazinyle, thiazolyle, dioxazinyle, thiadiazolylsulfonyle portant chacun, le cas échéant, un substituant méthyle, éthyle, fluoro, chloro, bromo, trifluorométhyle ou phényle ;
ou bien un groupement 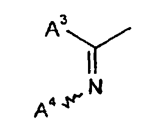 dans lequel
A³ est l'hydrogène ou un reste méthyle et
A⁴ est un reste hydroxy, méthoxy, éthoxy, amino, méthylamino, méthyle, phényle ou benzyle,
Z¹ représente une liaison simple, l'oxygène, le soufre ou un groupement 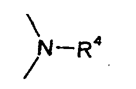 dans lequel
R⁴ est un reste méthyle, éthyle, n-propyle, isopropyle, n-butyle, isobutyle, sec.-butyle ou tertio-buyle ou forment conjointement avec R² et l'atome d'azote auquel ils sont liés un noyau hétérocyclique pentagonal ou hexagonal éventuellement substitué.

6. Compositions contenant des diluants et/ou des supports ainsi que, le cas échéant, des agents tensio-actifs, **caractérisées par** une teneur en au moins un composé de formule (I) suivant la revendication 1.

7. Procédé pour combattre des parasites, **caractérisé en ce qu'**on fait agir des composés de formule (I) suivant la revendication 1 ou des compositions suivant la revendication 6 sur les parasites et/ou sur leur milieu.

8. Utilisation de composés de formule (I) suivant les revendications 1 à 5 ou d'une composition suivant la revendication 6 pour combattre des parasites.

9. Procédé de préparation de compositions, **caractérisé en ce qu'**on mélange des composés de formule (I) suivant les revendications 1 à 5 avec des diluants et/ou des supports et/ou des agents tensio-actifs.

10. Procédé de production de composés de formule (I) tel que défini dans la revendication 1, **caractérisé en ce qu'**on fait réagir des carboxamides de formule générale (II) 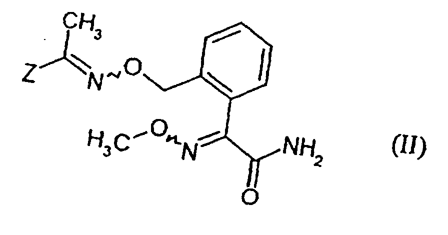 dans laquelle
Z a la définition indiquée ci-dessus, avec un halogénure d'acide carboxylique de formule (III) 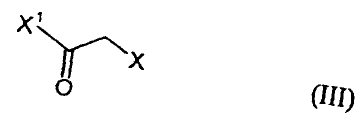
dans laquelle
X a la définition indiquée ci-dessus et
X¹ est un halogène, ou bien
avec un anhydride d'acide carboxylique de formule (IV) 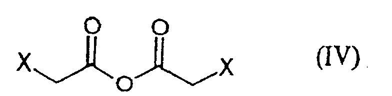 dans laquelle
X a la définition indiquée ci-dessus,
dans les deux cas en présence d'un diluant et dans les deux cas en présence d'un accepteur d'acide.
